Europäisches Patentamt

European Patent Office

Office européen des brevets

(19)

(11) EP 0 803 500 A1

(12) DEMANDE DE BREVET EUROPEEN

(43) Date de publication:
29.10.1997 Bulletin 1997/44

(51) Int Cl.$^6$: C07C 303/44, C07C 309/80

(21) Numéro de dépôt: 97400753.6

(22) Date de dépôt: 02.04.1997

(84) Etats contractants désignés:
AT BE CH DE DK ES FI FR GB IE IT LI LU NL SE

(30) Priorité: 24.04.1996 FR 9605170

(71) Demandeur: ELF ATOCHEM S.A.
92800 Puteaux (FR)

(72) Inventeurs:
• Ollivier, Jean
64260 Arudy (FR)

• Commarieu, Annie
982400 Courbevoie (FR)

(74) Mandataire: Leboulenger, Jean et al
Elf Atochem S.A.,
Département Propriété Industrielle,
Cedex 42 - La Défense 10
92091 Paris la Défense (FR)

(54) Procédé de purification d'un chlorure d'alcanesulfonyle et de l'acide alcanesulfonique correspondant

(57) Le procédé de purification d'un chlorure d'alcanesulfonyle (CAS) pour en diminuer la teneur en sulfates consiste à laver le chlorure d'alcanesulfonyle par de l'eau ou par une solution aqueuse d'un acide ayant un pH compris entre -0,57 et 7, puis à séparer ensuite le chlorure d'alcanesulfonyle ainsi purifié, de la phase aqueuse.

Ce CAS purifié conduit par hydrolyse totale à de l'acide alcanesulfonique à faible teneur en acide sulfurique.

Printed by Jouve, 75001 PARIS (FR)

## Description

La présente invention concerne la purification d'un chlorure d'alcanesulfonyle (CAS). Elle a plus particulièrement pour objet de réduire considérablement la teneur en acide sulfurique contenu dans l'acide alcanesulfonique (AAS) obtenu ultérieurement par hydrolyse dudit chlorure d'alcanesulfonyle.

Par l'expression "alcane" on entend les radicaux alcane inférieur ayant un nombre de carbone allant de 1 à 4, notamment les radicaux méthyle, éthyle, n-propyle, n-butyle.

Il est connu que pour les applications électrochimiques, ou pour les applications en traitement de surface de l'acide méthanesulfonique, la présence d'acide sulfurique est à éviter. En effet, en présence de plomb, du sulfate de plomb peut précipiter et limiter les performances de l'acide méthanesulfonique. La spécification commerciale pour ce genre d'application de l'acide méthanesulfonique est d'ailleurs telle que la teneur en acide sulfurique doit être inférieure à 350 p.p.m.

Il est déjà connu de purifier l'acide méthane sulfonique par précipitation ou par distillation.

-   Dans la méthode par précipitation, on utilise le fait que les sulfates de plomb ou de baryum sont insolubles dans l'acide méthanesulfonique ; cependant, le plomb ou le baryum qui subsiste dans l'acide méthanesulfonique après le traitement sont des contaminants indésirables.
-   Dans la méthode par distillation, on utilise le fait que la température d'ébullition de l'acide sulfurique est supérieure à celle de l'acide méthanesulfonique. Comme il s'agit d'éliminer des traces d'acide sulfurique dans l'acide méthanesulfonique, il faudrait en fait distiller l'acide méthanesulfonique du mélange acide méthanesulfonique/acide sulfurique. Non seulement cette solution n'est pas économiquement intéressante, mais encore elle nécessite un appareillage particulier (cf. brevet US 4 938 846), pour éviter la dégradation thermique de l'acide méthanesulfonique. L'usage montre qu'il est difficile de descendre en dessous de 1 000 p.p.m. d'acide sulfurique dans l'acide méthanesulfonique, alors que la spécification est à 350 p.p.m. d'acide sulfurique pour l'acide méthanesulfonique à 70 % en poids dans de l'eau.

Par ailleurs, le brevet US 4,549,993 enseigne un procédé de purification de chlorure d'alcanesulfonyle par lavage par une solution aqueuse d'acide chlorhydrique contenant au moins environ 18 % en poids de HCl par rapport au poids de la solution, puis en rectifiant le chlorure d'alcanesulfonyle séparé de la solution aqueuse à une température non supérieure à environ 70°C, sous pression réduite non supérieure à 500 torr et sous balayage d'un gaz inerte. De préférence, la concentration en HCl est de 30 à 36 % en poids pour réduire d'une manière significative l'hydrolyse du chlorure de méthanesulfonyle (CMS), le but technique étant d'obtenir du CMS sans acide méthanesulfonique, le problème des sulfates et en particulier de l'acide sulfurique n'étant pas abordé.

La Société déposante a maintenant trouvé que le problème de la présence d'acide sulfurique dans l'acide alcanesulfonique (AAS) peut être résolu par le traitement des chlorures alcanesulfonyles (CAS) par de l'eau ou par une solution aqueuse d'un acide ayant un pH compris entre -0,57 et 7, ce qui conduit à un CAS purifié, ce dernier pouvant être recueilli comme produit intermédiaire avant d'être soumis à une hydrolyse totale par de l'eau à température élevée pour donner l'acide alcanesulfonique (AAS) purifié.

Dans la suite de l'exposé, on appelle sulfate tout composé qui est présent en tant qu'impureté dans le chlorure d'alcanesulfonyle et qui, en présence d'eau, conduit à l'acide sulfurique $SO_4^{2-},2H^+$. Dans le cadre de cette définition, entrent par exemple, les composés $SO_4^{2-}$,contrecations, l'acide sulfurique lui-même, et les précurseurs de l'acide sulfurique, tels que notamment le chlorure de sulfuryle, $SO_3$, les esters alkyles inférieurs en $C_1$ à $C_4$ de l'acide chlorosulfonique.

La présente invention a donc d'abord pour objet un procédé de purification d'un chlorure d'alcanesulfonyle (CAS) pour en diminuer la teneur en sulfates, caractérisé en ce que l'on traite ledit chlorure en le mettant en contact avec un liquide de lavage choisi parmi l'eau ou une solution aqueuse d'un acide ayant un pH compris entre -0,57 et 7, puis en séparant ensuite le chlorure d'alcanesulfonyle du liquide de lavage, ce dernier ayant solubilisé la plus grande partie des sulfates en provoquant ainsi une diminution de la teneur en sulfates du chlorure d'alcanesulfonyle traité qui se trouve ainsi purifié.

De préférence, l'eau a un pH d'environ 7 ou bien la solution aqueuse d'un acide a un pH voisin de 7. Par exemple, de l'eau ayant dissous du dioxyde de carbone ($CO_2$) présent dans l'air ambiant ou encore de l'eau contenant des traces d'un acide. En effet, c'est avec de tels liquides de lavage qu'on observe la plus forte diminution en sulfates.

Avantageusement, le chlorure d'alcanesulfonyle est choisi parmi le chlorure de méthanesulfonyle (CMS), le chlorure d'éthanesulfonyle (CES), le chlorure de n-propanesulfonyle (CPS), le chlorure de n-butylesulfonyle (CBS).

Le traitement du chlorure d'alcanesulfonyle suivant le procédé de la présente invention peut s'effectuer en discontinu ou en continu. Suivant la solution technologique retenue on peut par exemple utiliser une agitation mécanique vigoureuse pour mettre en contact intime le chlorure d'alcanesulfonyle et l'eau ou la solution aqueuse qui sont essentiellement non-miscibles, ou encore utiliser un système mélangeur statique tel qu'une colonne verticale.

Dans le cas d'un traitement en discontinu le mélange CAS/eau ou CAS/solution aqueuse est vigoureusement agité pour augmenter momentanément la surface de contact à l'interface des deux phases liquides non-miscibles.

Avantageusement, la durée de cette agitation peut être choisie entre quelques secondes et 1 heure en fonction de la température du milieu agité et de préférence de 3 à 5 minutes, à la température de 20°C. Après la fin de l'agitation, on laisse décanter et on récupère la phase organique constituée par le CAS purifié.

Dans le cas d'un traitement en continu, l'appareillage comprend un mélangeur qui assure la mise en contact CAS/eau ou CAS/solution aqueuse et un décanteur dimensionné de telle sorte qu'il permette la séparation de la phase organique et de la phase aqueuse.

Avantageusement, le liquide de lavage est mis en oeuvre en une quantité en poids allant de 1 % à 50 % du poids du CAS à purifier. De préférence, la quantité de liquide de lavage est de 3% à 7% du poids du CAS à purifier.

Avantageusement, la mise en contact liquide de lavage/CAS se fait à une température des phases allant de 0°C à 50°C.

De préférence, cette température est de 10°C à 20°C pour ne pas induire des coûts d'exploitation élevés ni ne provoquer d'hydrolyse notable du CAS.

Avantageusement, la solution aqueuse d'un acide comprend de l'acide chlorhydrique à une teneur en poids inférieure à 18 % par rapport au poids total de cette solution aqueuse, assurant ainsi un pH supérieur à -0,57 et inférieur à 7.

La présente invention a également pour objet un procédé de fabrication d'acide alcanesulfonique à faible teneur en acide sulfurique, caractérisé en ce que l'on pratique l'hydrolyse totale du chlorure d'alcanesulfonyle purifié au préalable suivant le procédé ci-dessus avec éventuellement ses variantes.

L'acide alcanesulfonique (AAS) à faible teneur en acide sulfurique peut être notamment l'acide méthanesulfonique (AMS), l'acide éthanesulfonique (AES), l'acide n-propanesulfonique (APS), l'acide n-butanesulfonique (ABS) .

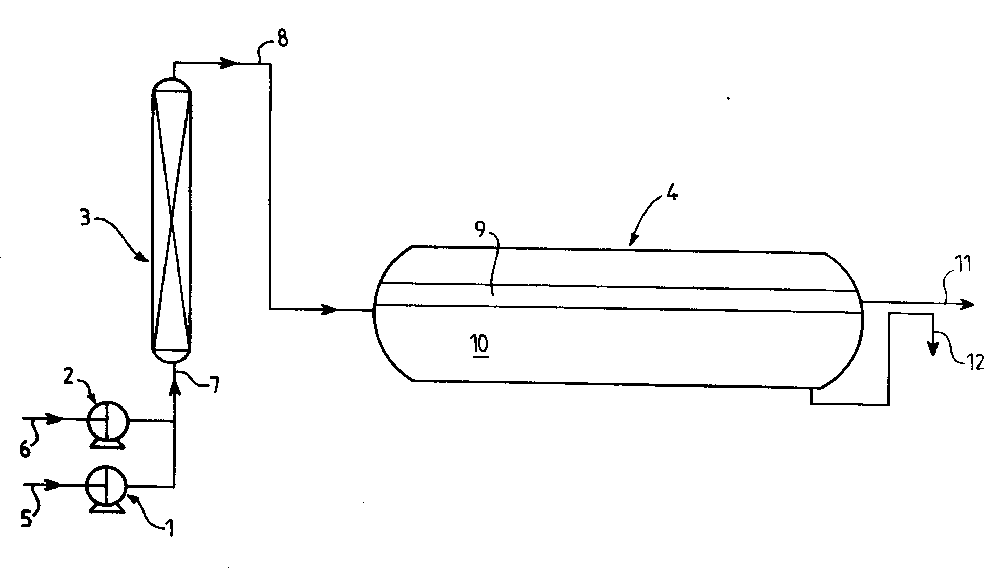

En plus de la description qui précède, les exemples suivants illustrent la présente invention avec notamment la figure unique qui représente schématiquement un appareillage conçu pour la mise en oeuvre du procédé de purification en continu d'un CAS.

**Partie expérimentale**

La teneur en sulfates dans les CAS comprend, l'anion sulfate lui-même $SO_4^{2-}$ ainsi que tout précurseur qui par hydrolyse conduit à l'anion sulfate $SO_4^{2-}$. La teneur en sulfates est toujours rapportée au poids de $SO_4^{2-}$ obtenu après hydrolyse totale du CAS initial et après l'hydrolyse totale du CAS purifié. Les hydrolyses sont réalisées à ébullition d'un mélange eau (20 g)/CAS (0,5 g) pendant au moins 3 minutes.

La différence de valeurs entre ces deux teneurs montre l'efficacité de la purification par contact avec le liquide de lavage.

La teneur en ions sulfates $SO_4^{2-}$ est mesuré par gravimétrie ou par chromatographie ionique des échantillons d'acides alcanesulfoniques obtenus par hydrolyse totale.

**Exemples 1 et 2**

A la température de 20°C, on introduit du CMS et 5 ou 10 % en poids d'eau bi-permutée (eau pure ne comportant pas d'ions d'une concentration totale supérieure ou égale à 0,5 p.p.m.) par rapport au poids total du mélange CMS/eau, dans une ampoule à décanter et on agite vigoureusement pendant 3 minutes. Après décantation, d'une durée de 24 heures, les phases CMS et aqueuses sont recueillies, pesées et analysées.

Les résultats obtenus figurent dans le tableau I ci-après.

TABLEAU I

|  |  | EXEMPLE 1 | EXEMPLE 2 |
|---|---|---|---|
| CMS initial | Masse en g | 95 | 90 |
|  | $SO_4^{2-}$ p.p.m. | 815 | 815 |
| Eau pure bi-permutée | Masse en g | 5 | 10 |
|  | $SO_4^{2-}$ en p.p.m. | <0,5 | <0,5 |
| CMS final | Masse en g | 97,3 | 92,7 |
|  | $SO_4^{2-}$ p.p.m. | 42 | 41 |
| Phase aqueuse finale | Masse en g | 3,2 | 8,7 |
|  | $SO_4^{2-}$ en p.p.m. | 1160 | 520 |

**Exemple comparatif 3**

Le mode opératoire est identique à celui de l'exemple 1 sauf que les 5 g d'eau pure sont remplacés par 5 g d'HCl à 33% en poids ayant un taux de sulfate inférieur à 8 p.p.m., valeur mesurée par les méthodes ci-dessus.

Après 5 heures de durée de décantation de la phase aqueuse et de la phase CMS, cette dernière a une teneur de 183 p.p.m. en $SO_4^{2-}$ rapportée au CMS.

Si l'on prolonge la durée de décantation à 24 heures, la teneur en $SO_4^{2-}$ rapportée au CMS tombe à 130 p.p.m.

**Exemple comparatif 4**

Le mode opératoire est identique à celui de l'exemple 1 sauf que les 5 g d'eau sont remplacés par 5 g d' HCl à 18% ayant un taux de sulfates inférieur à 8 p.p.m.

Après 5 heures de durée de décantation le CMS purifié contient 342 p.p.m. de $SO_4^{2-}$ rapporté au CMS.

Après 24 heures de décantation, ce taux tombe à 135 p.p.m.

**Exemple 5**

Le mode opératoire est identique à celui de l'exemple comparatif 3 ou 4, sauf que l'HCl à 33% ou 18% est remplacé par de l'HCl à 10% en poids d'une teneur en sulfates inférieure à 8 p.p.m.

Après 5 heures de durée de décantation le CMS purifié contient 111 p.p.m. de $SO_4^{2-}$ rapporté au CMS.

Après 24 heures de décantation, ce taux tombe à 100 p.p.m.

Les exemples 1 à 5 montrent que l'eau conduit à un meilleur résultat car à une plus faible teneur résiduelle en sulfates que ne le fait l'HCl à 10%, 18%, ou 33%.

L'HCl à 10% conduit à des résultats légèrement supérieurs à ceux obtenus par de l'HCl à 18 ou 33%.

**Exemple 6**

Le traitement est réalisé en discontinu.

On introduit 100g de CMS contenant 950 p.p.m. de sulfates ramenés en équivalent pondéral $SO_4^{2-}$ dans une ampoule à décanter avec 5 g d'eau distillée. Le mélange est ensuite agité énergiquement pendant 30 secondes, puis on laisse décanter pendant 5 heures. Au terme de cette durée le CMS est séparé de la phase aqueuse, puis analysé de manière habituelle. La teneur finale en $SO_4^{2-}$ dans l'AMS rapporté au CMS est de 40 p.p.m.

**Exemple 7**

Le mode opératoire est identique à l'exemple 1, sauf que le CMS est remplacé par le chlorure d'éthanesulfonyle (CES) contenant initialement 432 p.p.m. de sulfates calculés en équivalent pondéral $SO_4^{2-}$.

Après décantation, d'une durée de 24 heures, le CES purifié montre une teneur finale en $SO_4^{2-}$ rapporté au CES de 30 p.p.m.

**Exemple 8**

Le mode opératoire est identique à l'exemple 1, sauf que le CMS est remplacé par le chlorure de propanesulfonyle (CPS) contenant initialement 650 p.p.m. de sulfates calculés en équivalent pondéral $SO_4^{2-}$.

Après décantation, d'une durée de 24 heures, le CPS montre une teneur finale en $SO_4^{2-}$ rapporté au CPS de 25 p.p.m.

**Exemples 9 à 14**

Le traitement est réalisé en continu dans l'appareillage représenté sur la figure unique. Cet appareillage comporte une pompe doseuse 1 d'alimentation en eau 5 et une pompe doseuse 2 d'alimentation en CMS 6 d'un conduit commun 7 débouchant au pied d'une colonne verticale 3 faisant office de mélangeur statique. Cette colonne 3 mesure 400 mm de hauteur pour un diamètre intérieur de 10 mm (volume utile de 19 ml) et est garnie d'anneaux de verre de 4 mm de diamètre extérieur. Le mélange de CMS et de phase aqueuse sort en tête de colonne par le conduit 8 débouchant dans l'une des deux extrémités latérales d'un décanteur 4 horizontal tubulaire de 350 mm de long et de 100 mm de diamètre intérieur. La phase aqueuse 9 et le CMS sont maintenus à niveau constant dans le décanteur 4 à l'aide d'un conduit 11 de prélèvement de la phase aqueuse débouchant à l'autre extrémité du décanteur 4 et par un conduit 12 de prélèvement du CMS purifié débouchant sur le fond inférieur du décanteur pour soutirage du CMS plus dense que la phase aqueuse.

Les temps de séjour du CMS dans le décanteur peuvent varier de 4,5 à 5,5 heures et celui de l'eau de 3 à 59 heures. Les résultats obtenus figurent dans le tableau II ci-après.

TABLEAU II

| Exemples | Teneur initiale $SO_4^{2-}$ dans le CMS (p. p.m.) | $H_2O$ % en poids par rapport au poids total $H_2O$ et CMS | Température (°C) | Temps de séjour | | | Teneur finale en $SO_4^{2-}$ dans le CMS purifié (p.p.m.) |
|---|---|---|---|---|---|---|---|
| | | | | Mélangeur (min.) | CMS dans le décanteur (h) | Phase aqueuse dans le décanteur (h) | |
| 9 | 747 | 3,5 | 19 | 15 | 5,5 | 19 | 129 |
| 10 | 950 | 4 | 19 | 4 | 5 | 59 | 114 |
| 11 | 747 | 5,9 | 30 | 4 | 4,5 | 16 | 85 |
| 12 | 403 | 7,2 | 4 | 4 | 5 | 12 | 143 |
| 13 | 950 | 9,3 | 19 | 4 | 5,5 | 25 | 126 |
| 14 | 512 | 12,2 | 19 | 3,5 | 5 | 3 | 181 |

## Revendications

1. Procédé de purification d'un chlorure d'alcanesulfonyle (CAS) pour en diminuer la teneur en sulfates, caractérisé en ce que l'on traite ledit chlorure en le mettant en contact avec un liquide de lavage choisi parmi l'eau ou une solution aqueuse d'un acide ayant un pH compris entre -0,57 et 7, puis en séparant ensuite le chlorure d'alcanesulfonyle du liquide de lavage, ce dernier ayant solubilisé la plus grande partie des sulfates en provoquant ainsi une diminution de la teneur en sulfates du chlorure d'alcanesulfonyle traité et qui se trouve ainsi purifié.

2. Procédé suivant la revendication 1, caractérisé en ce que l'eau a un pH d'environ 7.

3. Procédé suivant la revendication 1, caractérisé en ce que la solution aqueuse d'un acide a un pH voisin de 7.

4. Procédé suivant l'une des revendications 1 à 3, caractérisé en ce que le chlorure d'alcanesulfonyle est choisi parmi le chlorure de méthanesulfonyle (CMS), le chlorure d'éthanesulfonyle (CES), le chlorure de n-propanesulfonyle (CPS), le chlorure de n-butylesulfonyle (CBS).

5. Procédé suivant l'une des revendications 1 à 4, caractérisé en ce que le liquide de lavage est mis en oeuvre en une quantité en poids allant de 1 % à 50 % du poids du chlorure d'alcanesulfonyle à purifier.

6. Procédé suivant la revendication 5, caractérisé en ce que ladite quantité est de 3 à 7 % du poids du chlorure d'alcanesulfonyle à purifier.

7. Procédé suivant l'une des revendications 1 à 6, caractérisé en ce que la mise en contact liquide de lavage/chlorure d'alcanesulfonyle se fait à une température des phases allant de 0°C à 50°C.

8. Procédé suivant la revendication 7, caractérisé en ce que ladite température est de 10 à 20°C.

9. Procédé suivant l'une des revendications 1 à 8, caractérisé en ce que la solution aqueuse d'un acide comprend de l'acide chlorhydrique à une teneur en poids inférieure à 18 % en poids par rapport au poids total de cette solution aqueuse, assurant ainsi un pH supérieur à -0,57 et inférieur à 7.

10. Procédé de fabrication d'acide alcanesulfonique à faible teneur en acide sulfurique, caractérisé en ce que l'on pratique l'hydrolyse totale du chlorure d'alcanesulfonyle purifié au préalable par le procédé de l'une des revendications 1 à 8.

Office européen
des brevets

**RAPPORT DE RECHERCHE EUROPEENNE**

Numero de la demande

EP 97 40 0753

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int.Cl.6) |
|---|---|---|---|
| A | DE 11 19 855 C (FARBENFABRIKEN BAYER AKTIENGESELLSCHAFT) <br> * revendication 1 * <br> --- | 1 | C07C303/44 <br> C07C309/80 |
| A | DE 11 03 323 C (FARBENFABRIKEN BAYER AKTIENGESELLSCHAFT) <br> * revendication 1 * <br> --- | 1 | |
| A | EP 0 373 304 A (PENNWALT CORPORATION) <br> * revendication 1 * <br> --- | 1 | |
| D,A | US 4 549 993 A (P.J. MCELLIGOTT, JR.) <br> * revendications 1,2 * <br> ----- | 1 | |

**DOMAINES TECHNIQUES RECHERCHES (Int.Cl.6)**

C07C

Le présent rapport a été établi pour toutes les revendications

| Lieu de la recherche | Date d'achèvement de la recherche | Examinateur |
|---|---|---|
| BERLIN | 16 Juillet 1997 | Kapteyn, H |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

.......................................................................

& : membre de la même famille, document correspondant

EPO FORM 1503 03.82 (P04C02)